# EUROPEAN PATENT APPLICATION

(11) **EP 2 255 813 A1**
(43) Date of publication of application: **01.12.2010**
(21) Application number: 09394013.8
(22) Date of filing: 29.05.2009
(51) Int. Cl.: A61K 31/575, A61P 1/12

(54) **Derivatives of ursodeoxycholic acid for the treatment of diarrhoea**

(71) Applicant: The Royal College of Surgeons in Ireland, Dublin 2 (IE)
(72) Inventor: The designation of the inventor has not yet been filed
(74) Representative: Purdy, Hugh Barry

(57) **Abstract**

A method for the treatment or prevention of diarrhoea (or condition characterised by diarrhoea) in an individual comprises a step of administering a therapeutically effective amount of metabolically stable UDCA to the individual.

## Description

### Introduction

The invention relates to a method for the treatment or prevention of dysregulated fluid transport into the intestine. In particular, the invention relates to a method of treatment or prevention of a specific clinical manifestation of such dysregulated fluid transport, namely diarrhoea.

Epithelial cells line the surface of the intestinal tract and are responsible for regulating the movement of fluid to and from the body. Fluid movement is driven by the active transport of ions and the establishment of osmotic gradients and in the intestine it is the secretion of Cl⁻ ions that is the primary driving force for fluid secretion. Cl⁻ ion secretion is promoted by immune cell mediators and neurotransmitters that elevate levels of intracellular 2^{nd} messengers, most notably, Ca²⁺ and cAMP. Many disease conditions exist (e.g. inflammatory bowel disease, infectious disease, celiac disease, irritable bowel syndrome, bile acid mal-absorption) that are characterised by dysregulated intestinal epithelial and fluid transport with the clinical manifestation of diarrhoea. However, to date, there is still a lack of therapeutic options for directly targeting epithelial transport processes in treatment of such conditions.

Bile acids are synthesised in the liver and are classically known for their roles in facilitating the digestion and absorption of fats. Two biosynthetic pathways for bile acids exist in the liver leading to the formation of two primary bile acids, cholic acid (CA) and chenodeoxycholic acid (CDCA). Upon ingestion of a meal, gall bladder contraction causes bile to be released into the proximal small intestine where the conjugated bile acids aid in the digestion and absorption of fats. As they travel through the small intestine, bile acids are reabsorbed through specific re-uptake pathways and are recycled back to the liver to be re-used. However, with each cycle of this enterohepatic circulation a small proportion (∼ 2%) of bile enters into the colon. Once in the colon bile acids undergo extensive metabolism by resident bacteria to create secondary bile acids. CA is metabolized primarily to deoxycholic acid (DCA), while CDCA is metabolized first to ursodeoxycholic acid (UDCA) which is further metabolized to lithocholic acid (LCA).

In the colon bile acids are known to regulate a number of epithelial functions including ion and fluid transport. When present at high concentrations some bile acids, including DCA and CDCA, can promote Cl⁻ and fluid secretion, which results in the onset of diarrhoea associated with conditions of bile acid malabsorption. While CDCA is rapidly metabolised to UDCA and LCA in the colon there is little information in the literature regarding the effects of UDCA on colonic secretion and no information on LCA in this regard. However, one side effect that has been reported for UDCA is that of diarrhoea. Beuers U, et al., 1992.

It is an object of the invention to overcome at least one of the above problems.

### Statements of the Invention

The invention is based on the surprising finding that UDCA exerts anti-secretory effects on colonic epithelial cells. *In vivo,* UDCA is rapidly metabolised to lithocholic acid (LCA) in the colon. It has been found that LCA has opposing effects to UDCA, in that it increases epithelial secretory responsiveness. Thus, the invention relates to the use of a stable analogue of UDCA, which cannot be metabolised to LCA, as an anti-diarrhoeal therapy.

Accordingly, the invention broadly relates to a method for the treatment or prevention of diarrhoea (or diarrhoeal disease) in an individual comprising a step of administering to the individual a therapeutically effective amount of metabolically stable UDCA.

The invention also relates to a method for the treatment or prevention of dysregulated fluid transport in an individual comprising a step of administering to the individual a therapeutically effective amount of metabolically stable UDCA.

The invention also relates to a method for treatment / prevention of a disease characterised by dysregulated fluid transport in an individual comprising a step of administering to the individual a therapeutically effective amount of metabolically stable UDCA.

The invention also relates to the use of metabolically stable UDCA for the treatment of symptoms of dysregulated fluid transport, typically diarrhoea or diarrhoeal disease.

The invention also relates to the use of a metabolically stable UDCA as a medicament.

The invention also relates to a pharmaceutical composition comprising a metabolically stable UDCA and a pharmaceutical excipient.

### Definitions

In this specification the term "metabolically stable UDCA" should be taken to mean an analogue of UDCA that is resistant to conversion to LCA in vivo, and which has the ability to exert anti-secretory effects on colonic epithelial cells. Examples of metabolically stable UDCA will be well known to the person skilled in the art from the literature for example; modifications to position 6 and 7 of the steroid ring of the UDCA molecule or modifications to a side chain of the UDCA molecule; including modifications to the length of the side chain, modifications to the carboxy terminal of the side chain and modifications to the side chain that are capable of causing steric / electronic hindrance and so prevent dehydroxylation of 7OH to LCA. Roda, A. *et al.,* 1989, Roda, A. *et al.,* 1990 and Clerici, C. et al., 1992 teach that modifications to the side chain of the UDCA molecule that cause steric hindrance or electronic hindrance by way of such processes as hydroxylation, esterification, amidation and methylation can also alter colonic metabolism.

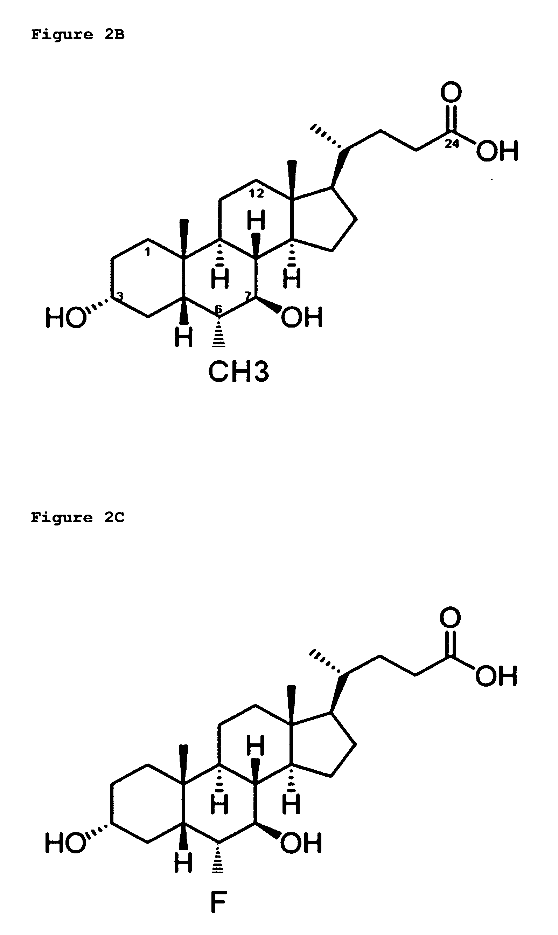

6-methyl-UDCA and 6-fluoro-UDCA are examples of metabolically stable UDCA compounds and their synthesis is described in Roda *et al*., 1994 and Roda et al., 1995 respectively (Figure 2B and Figure 2C). In both of these compounds, the 7-hydroxy substituent of UDCA (which is the target for the bacterial dehydroxylation enzymes during the conversion of UDCA to LCA) is stablilised by the modification of the 6C position on the steroid ring of the UDCA molecule which renders it resistant to dehydroxylation. While 6-methyl and 6-fluoro analogues are specifically disclosed, other modifications at the 6-position are also envisaged, for example 6-acyl or 6-halogen derivatives.

Another functional method for producing metabolically stable UDCA is the modification, by means of conjugation or sulfonation, of the carboxy terminal of the UDCA side chain to prevent dehydroxylation of the 7-OH group. Mikami, T. et al., 1993, describe the production of sulfonate analogues of UDCA that are metabolically stable.

In this specification, the term "treatment or prevention" should be taken to mean both prophylactic and symptomatic treatment of an individual, including an individual who already exhibits the symptoms of dysregulated fluid transport, or an individual who does not yet have the symptoms of dysregulated fluid transport but who has been diagnosed with a condition characterised by dysregulated fluid transport. Further, the term should be taken to include treatments which are effective in removing or stopping the symptoms and treatments which inhibit or ameliorate the symptoms.

In the specification, the term "individual" should be taken to mean a human, however it should also include higher mammals for which the therapy of the invention is practicable.

In this specification, the term "therapeutically effective amount" should be taken to mean an amount of metabolically stable UDCA which results in a clinically significant reduction or prevention of the symptoms of diarrhoea. Suitably, the metabolically stable UDCA is administered at a dose of between 1 microgram and 10 grams daily, preferably between 1 milligrams and 1 gram daily, more preferably between 500 milligrams and 1 gram daily.

In this specification, the term "disease or condition or pathology" characterized by dysregulated fluid transport should be taken to mean inflammatory bowel disease, infectious disease, celiac disease, irritable bowel syndrome, any diseases or conditions associated with bile acid malabsorption, gastrointestinal infections, and other diseases or conditions that result in the clinical manifestation of diarrhoea.

In this specification, the term "administering" should be taken to include any form of delivery that is capable of delivering the metabolically stable UDCA to colonic epithelial cells, including intravenous delivery, oral delivery, intramuscular delivery, intrathecal delivery, and inhaled delivery. Methods for achieving these means of delivery will be well known to those skilled in the art of drug delivery.

In this specification, the term "pharmaceutical composition" should be taken to mean compositions comprising a therapeutically effective amount of metabolically stable UDCA, and a pharmaceutically acceptable carrier or diluent. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the metabolically stable UDCA is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

### Brief Description of the Figures

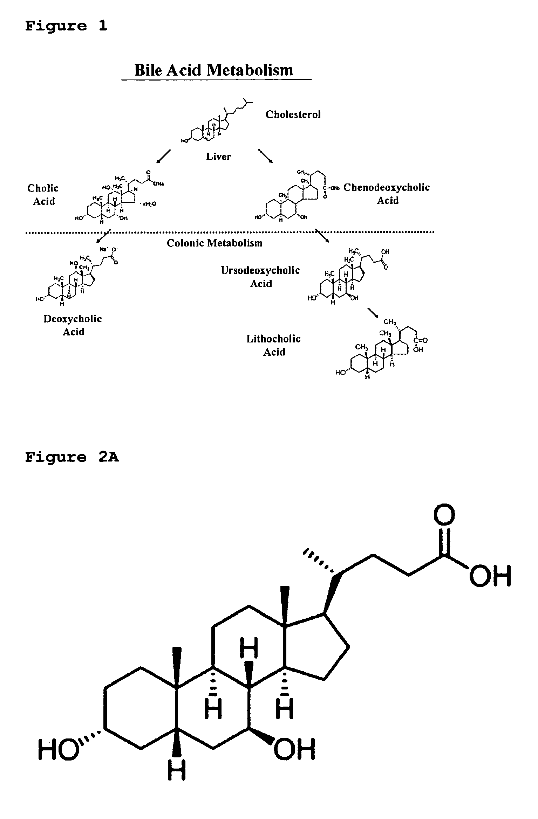

**Figure 1****:** Bile acid metabolism. The primary bile acids, chenodeoxycholic acid and cholic acid are synthesised in the liver. In the colon these bile acids are metabolised by resident bacteria to produce secondary bile acids, including deoxycholic acid, ursodeoxycholic acid and lithocholic acid.
**Figure 2A****:** Chemical structure UDCA
**Figure 2B****:** Chemical structure metabolically stable 6-methyl-UDCA
**Figure 2C****:** Chemical structure metabolically stable 6-fluoro-UDCA

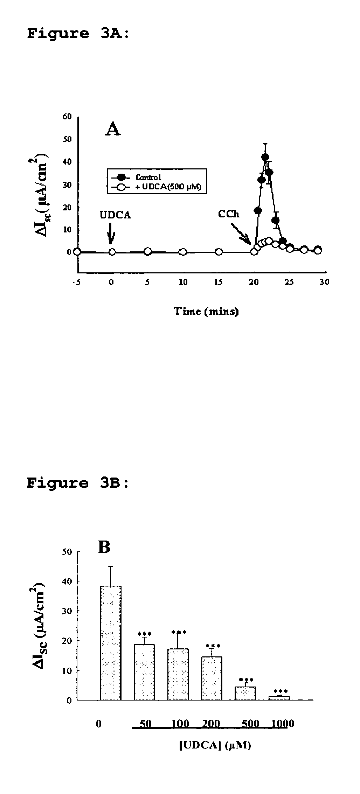

**Figure 3A****:** The effect of UDCA (500 µM) on basal short circuit current (I_{sc}) and on I_{sc} responses to the Ca²⁺-dependent secretagogue, carbachol (CCh) across cultured monolayers of T₈₄ cells. Cells were pretreated with UDCA for 20 min after which Cl⁻ secretory responses to CCh were measured as changes in I_{sc}. UDCA (500 µM), while having no effect on basal I_{sc} alone, practically abolished the ability of the cells to evoke secretory responses to CCh.
**Figure 3B****:** The effect of UDCA (50 µM - 1 mM) on I_{sc} responses to CCh in T₈₄ cells. Cells were pretreated with UDCA for 20 min after which maximal Cl⁻ secretory responses to CCh were measured. UDCA was found to significantly reduce responses to CCh in a concentration-dependent manner.

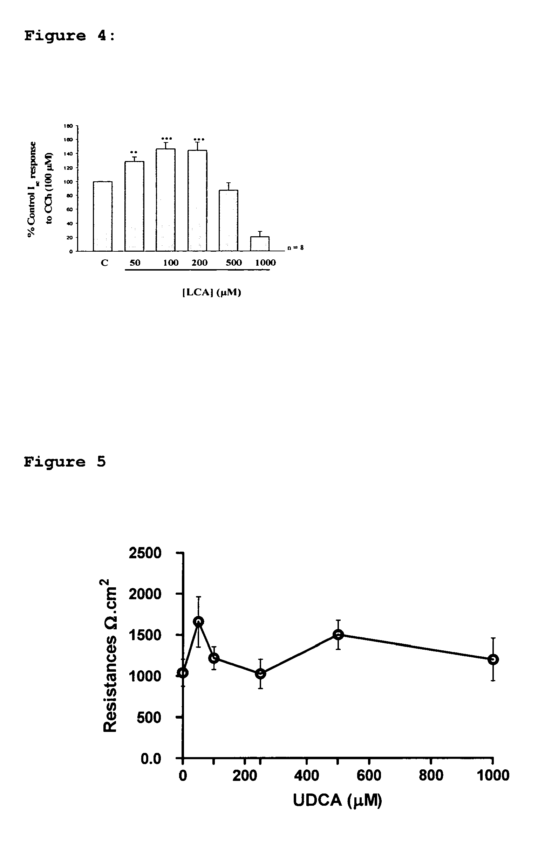

**Figure 4****:** The effect of LCA (50 µM - 1 mM) on I_{sc} responses to CCh in T₈₄ cells. Cells were pretreated with LCA for 20 min after which maximal Cl⁻ secretory responses to CCh were measured. Data are expressed as % control response to CCh. At low (50 µM - 200 µM) concentrations, LCA potentiated responses to CCh while at higher concentrations (> 500 µM), LCA was found to reduce responses to CCh. LCA alone was without effect on basal I_{sc}.
**Figure 5****:** UDCA does not effect transepithelial resistance at concentrations up to 1mM. Cells were treated with UDCA at the concentrations indicated and 20 mins later transepithelial resistance (TER) was measured using the EvOhm apparatus. UDCA did not alter TER at any concentration tested indicating it was without toxic effects.

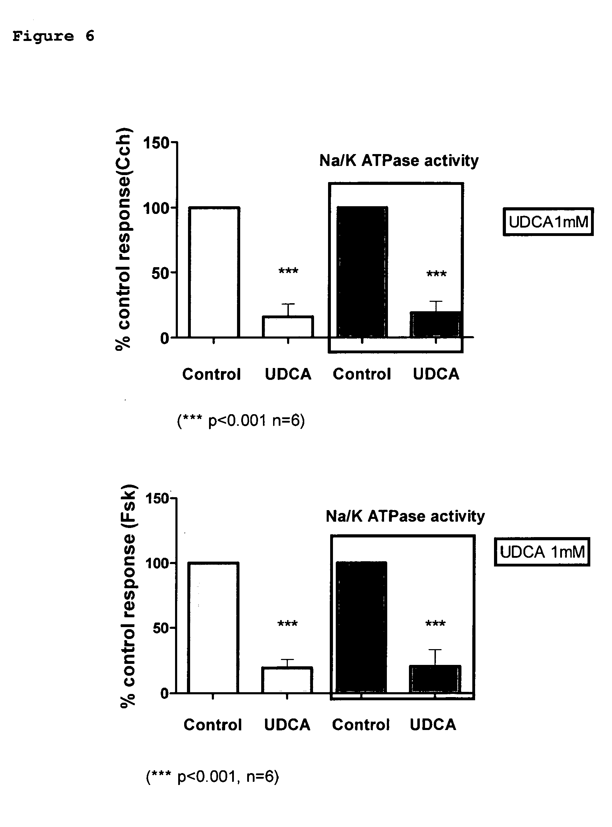

**Figure 6****:** UDCA inhibits Na⁺/K⁺ ATPase activity. Monolayers were apically permeabilised using amphotericin B (10 mM) and changes in Na+/K+ ATPase activity was measured as changes in ouabain-sensitive current. UDCA (1 mM) was found to attenuate sodium pump activity to a similar degree as its effects on transepithelial Cl⁻ secretion (n=6, ***p<0.001).

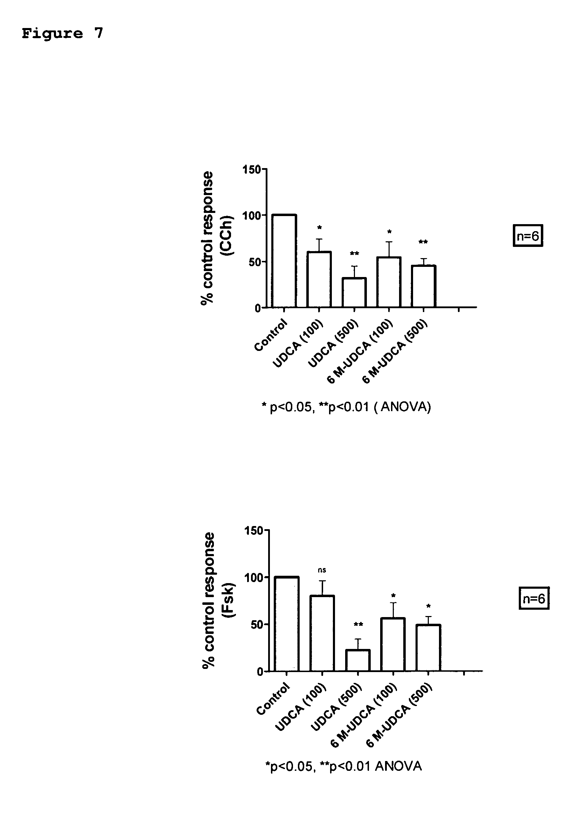

**Figure 7****:** Cells were treated with either UDCA or 6-methyl-UDCA (MUDCA) at concentrations of 100µM and 500µM for 15 minutes prior to stimulation with either the Ca⁺⁺ or c-AMP dependent agonists, CCh (100µM) and FSK (10µM), respectively. Similar to the parent compound, UDCA, the metabolically stable derivative, MUDCA, inhibited secretory responses to both CCh and FSK. Responses are expressed as % of control ΔI_{sc} responses to either CCh or FSK.

### Detailed Description of the Invention

**Materials and Methods:** Cultured T₈₄ epithelial cells were employed as a model of the colonic epithelium. T₈₄ cells were grown as monolayers on filter supports. When grown in this way T₈₄ cells retain many of the characteristics of native intestinal epithelium and are widely considered as being among the best reductionist models for analysing intestinal epithelial secretory responses. T₈₄ cell monolayers were mounted in Ussing chambers, and voltage-clamped to zero potential difference. Under these conditions, transepithelial ion transport can be measured as changes in short-circuit current (I_{sc}) and such changes in I_{sc} across T₈₄ cells have been established as wholly due to Cl⁻ ion secretion. After mounting the cells in Ussing chambers the cells were treated with either UDCA or LCA at different concentrations (50 µM - 1 mM) and changes in I_{sc} were monitored for 20 minutes. After this Cl⁻ secretory responses to the calcium-dependent secretagogue, carbachol (100µM) and FSK (10µM) were measured.

**Results:** Treatment of the T₈₄ cell monolayers with UDCA (500 µM) did not alter basal I_{sc} on its own. However, pretreatment with UDCA practically abolished the ability of the cells to evoke responses to CCh (Figure 3A). This effect of UDCA was concentration-dependent with antisecretory effects being apparent at concentrations as low as 50 µM (Figure 3B) . Similar to UDCA we found that LCA alone was without effect on basal Cl⁻ secretion (data not shown). However, pretreatment with LCA had contrasting effects on subsequent CCh-induced responses depending on its concentrations used. At low concentrations, LCA (50 - 200 µM) significantly potentiated subsequent responses to CCh, while at higher concentrations (> 500 µM) LCA was antisecretory (Figure 4). In further experiments we specifically examined the effects of UDCA on the activity of the Na⁺/K⁺ ATPase pump, which provides the driving force for Cl⁻ secretion to occur. We found that UDCA inhibited Na⁺/K⁺ ATPase activity to a similar degree as its effects on Cl⁻ secretion (Figure 6). Finally, the stable analogue of UDCA 6-methyl UDCA (MUDCA; 100 µM - 500 µM) mimicked the antisecretory effects of UDCA (Figure 7) .

**Discussion:** UDCA is formed in the colon by the action of bacteria which epimerise the 7-OH group of CDCA. Unlike its parent compound, CDCA which is prosecretory, this novel data shows that UDCA is an antisecretory bile acid. UDCA has been used in the therapy of liver disease for many years and is considered to be an exceptionally safe drug with few side effects. However, one side effect that has been reported for UDCA is that of diarrhoea. In light of this data showing that UDCA is antisecretory, the reported finding seems counterintuitive. However, the finding that LCA is rapidly formed from UDCA by bacterial-mediated dehydroxylation of the 7-OH group provides an explanation for these observations (Figure 1). This data shows that at low concentrations LCA increases epithelial responsiveness to secretory agonists, typified by CCh. Thus, while UDCA is antisecretory, such actions are not observed in vivo due to its rapid conversion to LCA in the colon. The data indicates that stable analogues of UDCA, which cannot be dehydroxylated to LCA, are effective anti-diarrhoeal agents.

The invention is not limited to the embodiments hereinbefore described which may be varied in construction and detail without departing from the spirit of the invention.

### References

Beuers U, Spengler U, Kruis W, Aydemir U, Wiebecke B, Heldwein W, Weinzierl M, Pape GR, Sauerbruch T, Paumgertner G. Ursodeoxyxholic acid for treatment of primary schlerosing cholangitis: a placebo-controlled trial. Hepatology (1992) 16: 707-714.
Clerici C, et al., Effect of intraduodenal administration of 23-methyl-UDCA diastereoisomers on bile flow in hamsters; Dig. Dis Sci, 1992, 37(5): 791-798
Mikami T, et al., Metabolism of sulfonate analogs of ursodeoxycholic acidand their effects on biliary bile acid composition in hamsters; J. Lipid Res. 34(3) 429-435
Roda A, et al., Structure activity relationship studies on natural and synthetic bile acid analogs; Dis Sci, 1989, 34(12) 24S-35S
Roda A, et al., Physicochemical and biological properties of natural and synthetic C-22 and C-23 hydroxylated bile acids; J. Lipid Res. 1990 31(2) 289-298
Roda A, et al., New 6-substituted bile acids; physic-chemical and biological properties of 6α-methyl urosdeoxycholic acid and 6-methyl-7-epicholic acid, J. Lipid Res. 1994 35 2268-2279
Roda A, et al., Metabolism, Pharmacokinetics, and Activity of a New 6-Fluoro Analogue of Ursodeoxycholic Acid in Rats and Hamsters, Gastroenterology, 1995, 108 1204-1214

## Claims

1. Use of a therapeutically effective amount of metabolically stable UCDA in the treatment or prevention of diarrhoea.

2. Use as claimed in Claim 1 in which the metabolically stable UDCA is 6-methyl-UDCA having the chemical structure:

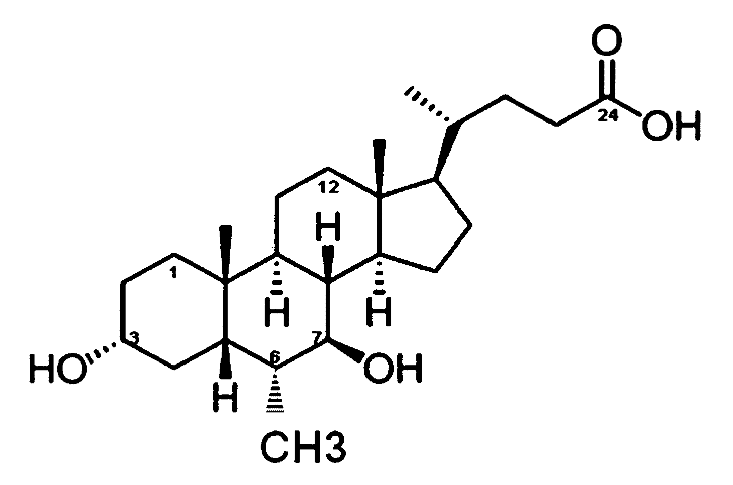

3. Use as claimed in Claim 1 in which the metabolically stable UDCA is 6-fluoro-UDCA having the chemical structure:

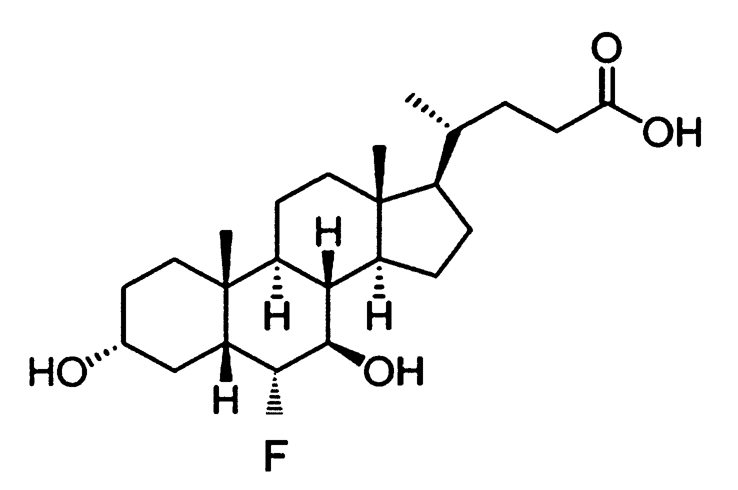

4. Use as claimed in Claim 1 in which the metabolically stable UDCA comprises UDCA that is modified at the 6- or 7- position on the steroid ring.

5. Use as claimed in Claim 4 in which the metabolically stable UDCA comprises 6-lower alkyl-UDCA or 6-halo-UDCA.

6. Use as claimed in Claim 5 in which the lower alkyl is a C1 to C4 alkyl group.

7. Use of a metabolically stable UDCA as a medicament.

8. A pharmaceutical composition comprising a metabolically stable UDCA and a pharmaceutical excipient.
